# EUROPEAN PATENT SPECIFICATION

(11) **EP 3 324 899 B1**
(45) Date of publication and mention of the grant of the patent: **30.03.2022**
(21) Application number: 16831095.1
(22) Date of filing: 21.07.2016
(51) Int. Cl.: A61F 5/01, A61F 5/04, A61F 5/042, A61F 5/05, A61F 5/058, A61F 13/04

(54) **QUICK DEPLOYMENT CAST**
SCHNELLEINSATZGUSS
PLÂTRE À DÉPLOIEMENT RAPIDE

(30) Priority: 21.07.2015 US 201514805460
(43) Date of publication of application: 30.05.2018
(73) Proprietor: Iron Horse Prime, LLC, Northbrook, IL 60062 (US)
(72) Inventor: ELSMO, Alan Clark, Northbrook, IL 60062 (US)
(74) Representative: Acapo AS
(86) International application number: PCT/US2016/043304
(87) International publication number: WO 2017/019443

(56) References cited:
- WO-A1-02/067832
- BE-A3- 1 017 372
- US-A- 3 930 496
- US-A- 4 019 506
- US-A- 4 483 332
- US-A- 4 483 332
- US-A- 4 498 467
- US-A- 4 502 479
- US-A- 4 996 979
- US-A- 5 318 504
- US-A1- 2009 171 356

## Description

### Technical Field

This disclosure relates to casts in medical applications in orthopedics, including specific applications for military field use and prevention of compartment syndrome. Utilizing a method and device along with an algorithm comparing normative and patient-specific data, the user and practitioner are provided with early detection for complications associated with immobilizing a traumatized limb. US4483332 discloses a quick deployment cast, comprising a bladder network (tubing) with a reactable substance therein, such as polymers and an input valve for a reaction fluid such as warm water for initiating the hardening of the polymers.

### Background of the Invention

The subject disclosure generally relates to casting, monitoring, alerting, modifying and removing casting materials. The invention is defined in the claims. Typically, application and removal of casts on a broken or sprained limb requires special tools, materials and expertise. These present challenges including superficial and cosmetic inconveniences, in addition to life-threatening and longer-term risks, such as lung cancers from chronic inhalation of fiberglass shavings. Additionally, trauma and compression of a limb through casting can lead to compartment syndrome which can require procedures that present significant risk and downside for the patient and orthopedic community.

### Brief Summary of Embodiments of the Invention

According to one embodiment of the disclosure a quick deployment cast includes a flexible outer sleeve fitted for a human appendage and a bladder network within the sleeve. A fluid capable of expanding and hardening, when in the presence of a curing agent, is stored within the bladder. A mechanism exposes the fluid to the curing agent.

According to another embodiment of the disclosure, a quick deployment cast includes a bladder network, a fluid, and a mechanism. The bladder network has one or multiple intersecting sets of hollow ducts. The fluid is stored within the bladder network and is capable of expanding and hardening when in the presence of a curing agent. The mechanism exposes the fluid to the curing agent.

Other features and aspects of the invention will become apparent from the following detailed description, taken in conjunction with the accompanying drawings, which illustrate, by way of example, the features in accordance with embodiments of the invention. The summary is not intended to limit the scope of the invention, which is defined solely by the claims attached hereto.

### Brief Description of the Drawings

The present invention, in accordance with one or more various embodiments, is described in detail with reference to the following figures. The drawings are provided for purposes of illustration only and merely depict typical or example embodiments of the invention. These drawings are provided to facilitate the reader's understanding of the invention and shall not be considered limiting of the breadth, scope, or applicability of the invention. It should be noted that for clarity and ease of illustration these drawings are not necessarily made to scale.

Some of the figures included herein illustrate various embodiments of the invention from different viewing angles. Although the accompanying descriptive text may refer to such views as "top," "bottom" or "side" views, such references are merely descriptive and do not imply or require that the invention be implemented or used in a particular spatial orientation unless explicitly stated otherwise.
Fig. 1 is a perspective view of the quick deployment cast in use in a military application;
Fig. 2 is a perspective view of the quick deployment cast;
Fig. 3 is a detail view of a check valve port of the quick deployment cast;
Fig. 4 is a detail view of a check valve port of the quick deployment cast with polyurethane gel deployed;
Fig. 5 is a perspective view of a check valve port of the quick deployment cast with polyurethane gel deployed;
Fig. 6 is a perspective view of the quick deployment cast with balloons deployed;
Fig. 7 is a detail view of the quick deployment cast with balloons deployed;
Fig. 8 is a perspective view of the quick deployment cast illustrating the bactericidal layer attached to the skin-side of the bladder and mimicking the helical and anti-helical pattern of the bladder network;
Fig. 9 is a perspective view of the quick deployment cast illustrating seams of the cast, capable of being disassembled via a zipper connection;
Fig. 10 is a perspective view of an application of the quick deployment cast;
Fig. 11 is top view of a sleeve of the quick deployment cast;
Fig. 12 is a rear view of the sleeve of the quick deployment cast;
Fig. 13 is a cut away view of the bladder network of the quick deployment cast with the surrounding sleeve;
Fig. 14 is a detail view of the bladder network of the quick deployment cast;
Fig. 15 is a perspective view of the quick deployment cast illustrating seams of the cast, capable of being disassembled via wires;
Figs. 16A-C are various perspective views of the quick deployment cast illustrating an expansion seam and an expansion zipper providing minimal expansion to relieve pressure while remaining in place and maintaining a degree of stability;
Fig. 17 is a system overview of the quick deployment cast for saving and communicating sensor data.

The figures are not intended to be exhaustive or to limit the invention to the precise form disclosed. It should be understood that the invention can be practiced with modification and alteration, and that the invention be limited only by the claims.

### Detailed Description of the Embodiments of the Invention

From time-to-time, the present invention is described herein in terms of example environments. Description in terms of these environments is provided to allow the various features and embodiments of the invention to be portrayed in the context of an exemplary application. After reading this description, it will become apparent to one of ordinary skill in the art how the invention can be implemented in different and alternative environments.

Unless defined otherwise, all technical and scientific terms used herein have the same meaning as is commonly understood by one of ordinary skill in the art to which this invention belongs.

The following terms are used throughout this document:
cast 10
check valve ports 15
release mechanism 18
sleeve 20
balloon ports 25
bladder tubes 30
polyurethane gel 35
sensors 40
balloons 45
graphene wires 55
wires 60
seams 65
zipper connection 66
expansion seams 67
expansion zipper 68
expansion material 69
brace 70
bilateral sleeves 75
processor 110
sensor module 140
memory module 150
communication module 160

The present invention is directed toward a cast 10 or splint or tourniquet for a broken or traumatized limb that may be quickly and easily deployed in the battlefield or any other environment that is remote from a hospital. The invention may also be used in a hospital or clinic environment. The quick deployment cast 10 may be used as a tourniquet, with desired pressure being selected, thereby allowing the device to expand to create greater tension/pressure against the limb.

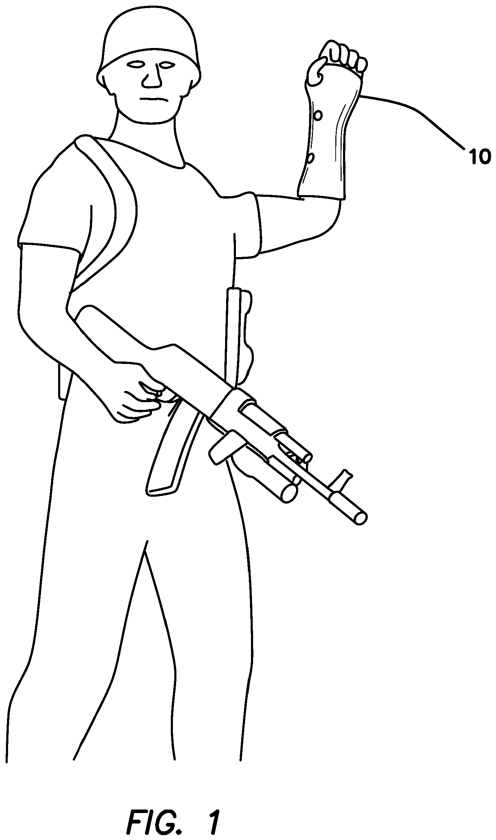

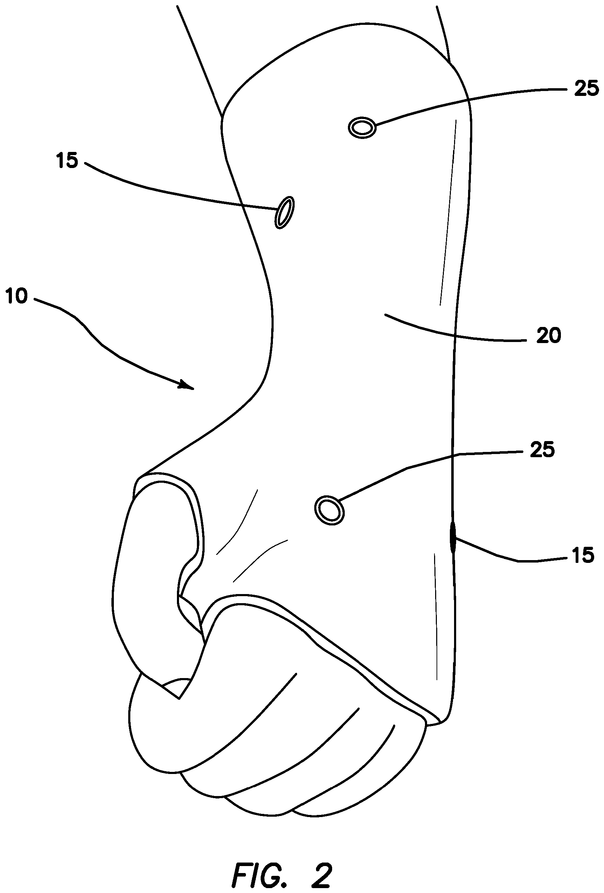

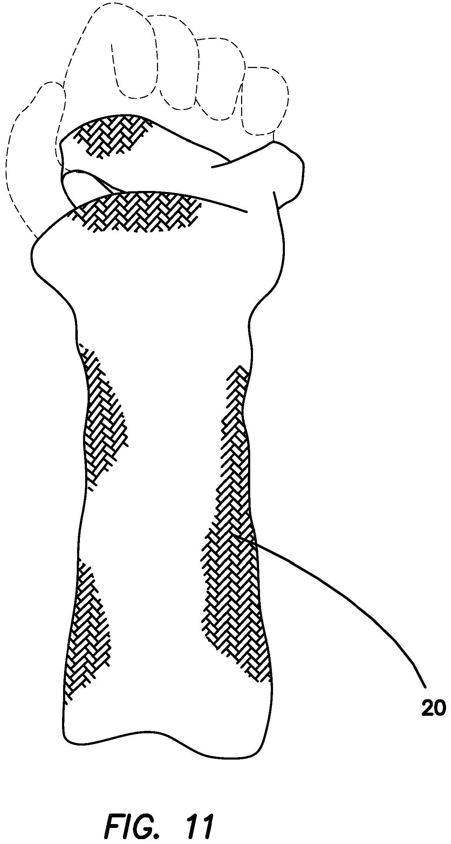

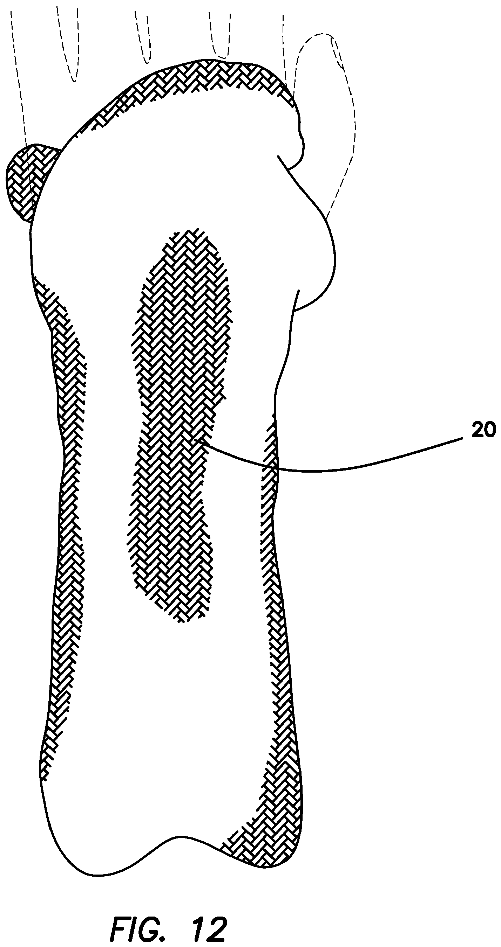

In a variant, referring to Figs. 1-5 and 10-12, the cast 10 comprises a sleeve 20 having a graphene fabric outer covering. The cast is pulled onto a whole or portion of a wearer's limb such as an arm or leg - similar to pulling on a sock. The cast 10 includes a number of check valve ports 15 and a number of balloon ports 25. The valves 15 have a release mechanism 18 that when actuated permit air to enter the valve 15 and enter bladder tubes 30. The cast 10 has a framework consisting of a web or network of bladder tubes 30 (which preferably are made of a graphene material) that is covered by the fabric outer covering, which is graphene or other fabric. The bladder network 30 may be oriented in any combination of patterns, including but not limited to double, triple or quadruple helical, with one set of tubes running parallel and another helical set intersecting the first set a various points.

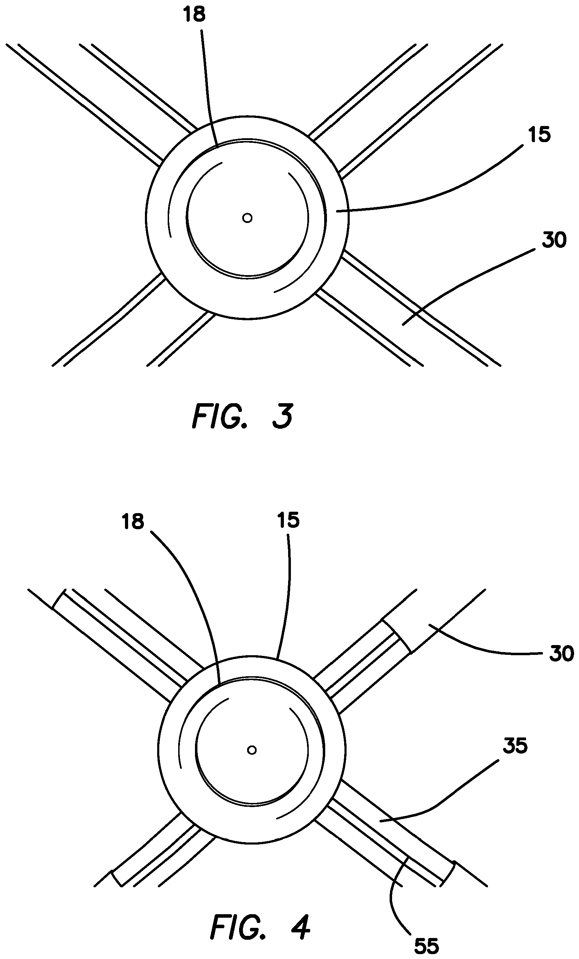

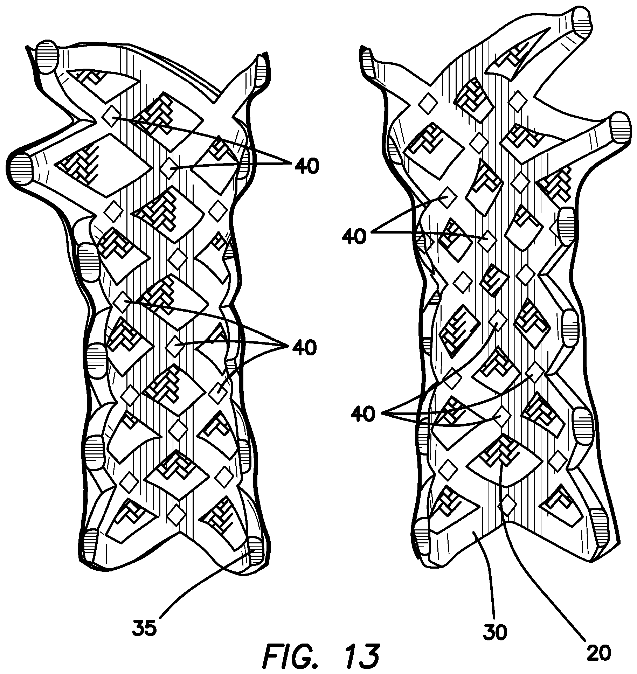

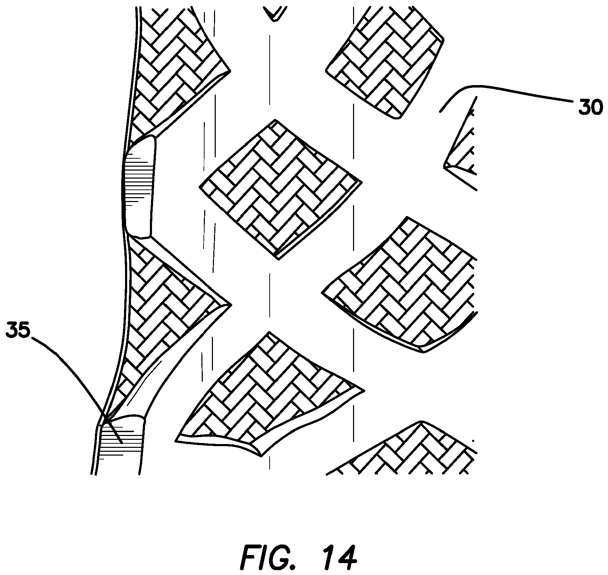

Referring also to Figs.13-14, the bladder tubes 30, which are in fluid communication with the check valve 15 and balloon ports 25, contain a polyurethane gel 35. The polyurethane gel 35 expands to a multiple of its initial volume (depending on desired properties like rigidity) when exposed to air or other curing agent. The expansion of the gel 35 may be, for example, but not limited to, 3 to 4 times its initial volume when exposed to air or other curing agent. Other expansion multiples are possible depending on available materials for the gel 35 and the intended use of the cast 10. Referring to Fig 3, when the cast 10 is stored in a state ready for use, the interiors of the tubes 30 containing the gel 35 are kept at near vacuum, with the check valves 15 closed, and the overall device compressed like a spring.

In an optional variant, a cellulose sponge is embedded in the bladder network 30, and the sponge is soaked in the polyurethane gel 35. In a variant, the sponge is a dehydrated, compressed cellulose sponge. The soaked sponge and polyurethane gel 35 are activated by water that is introduced from reservoirs atop the check valves 15 when the sleeve is pulled onto the limb, similar to the variant having polyurethane and graphene spires within the bladder. The bladder tubes 30, which are in fluid communication with the check valve 15 and balloon ports 25, contain the sponge soaked polyurethane gel 35, and the sponge-gel may expand to a multiple of 3 to 4 times (or more) its initial volume when exposed to water.

In yet another variant, the sponge contains compartmentalized moisture to be combined with and activate the polyurethane when the cast 10 is pulled on, with the stress of pulling on and stretching the cast 10 providing enough force to rupture the wall dividing the polyurethane and water, thereby actuating the curing process. The duration of the curing process depends on, for example, the type of polyurethane gel 35 used, the desired expansion of the gel 35, desired rigidity, ease of removal, and other factors. In some embodiments, the duration of the curing process may be adjusted.

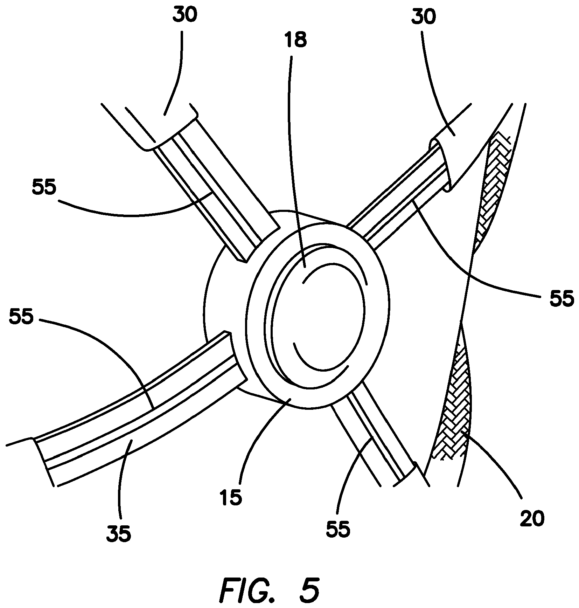

In a variant, referring to Figs. 4 and 5, when the cast 10 is pulled onto a wearer's arm, the check valves 15 open so that air enters the bladder tubes 30 and the polyurethane gel 35 expands and cures so that a rigid or semirigid cast 10 is formed. The check valves 15 are operable to open easily by, for example, but not limited to, a light actuation of the release mechanism 18 by the user, because the bladder tubes 30 are kept near vacuum pressure or a negative relative pressure compared to the atmosphere. Figs. 4-5, show the bladders 30 partially cut away for the purpose of illustrating the presence of the polyurethane gel 35 inside the bladders 30. Optionally, the valves 15 may be manually opened without use of the release mechanism 18.

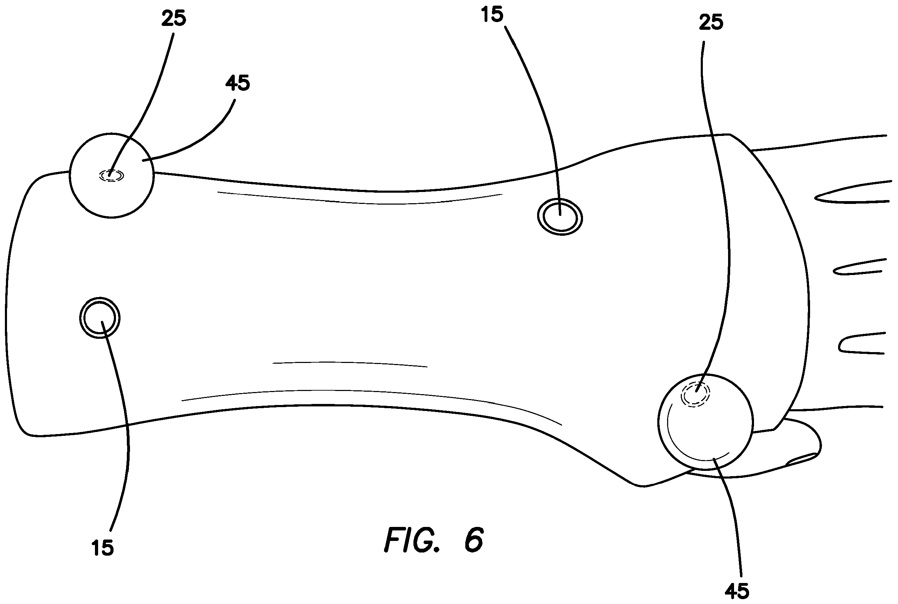

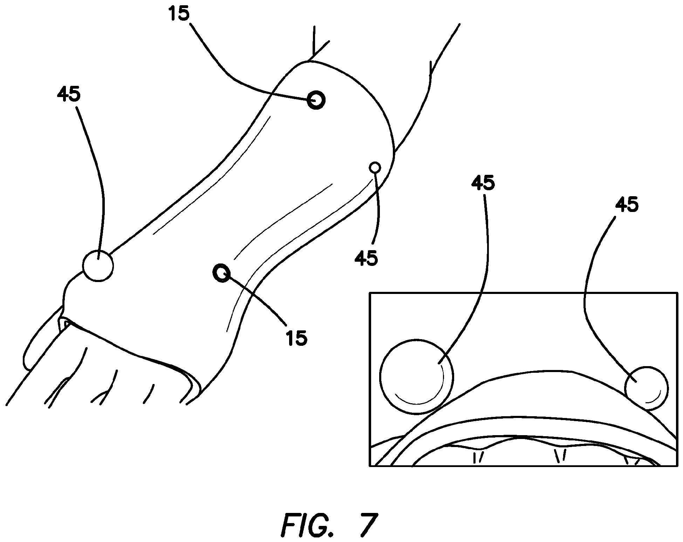

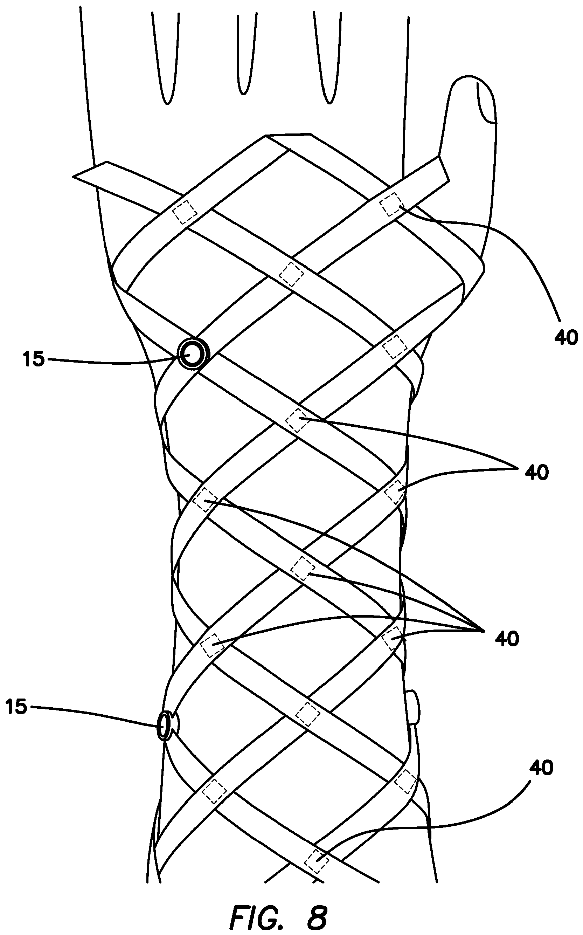

In another variant, referring to Fig. 8, the bladder tube framework may also contain sensors 40 for detecting tension or pressure, so that when the proper tension on the bladder tubes 30 is reached, the excess or surplus polyurethane may be directed into balloons 45 which are housed in balloon ports 25 as illustrated in Figs 6 and 7. After the polyurethane cures, the user may snap the balloons off of the cast 10 manually.

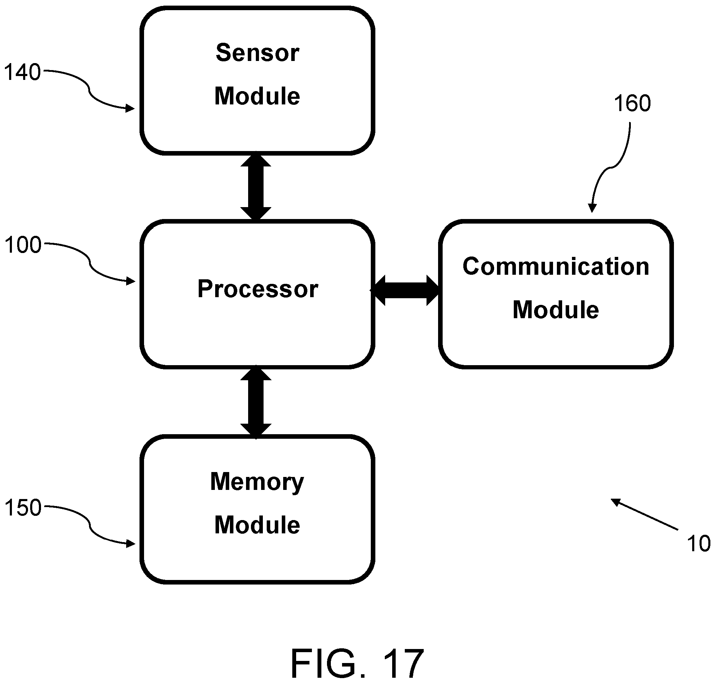

In yet another variant, the sensors 40 also monitor conditions of the cast 10 and the user's limb, such as the integrity, shape and pressure of the cast 10. Additionally, bio-sensors including, but not limited to pH, pulse, blood-flow, blood oxygen, enzymes, proteins and other compounds are monitored. The cast 10 communicates this information (see description of Fig. 17 for more detail) to a portable device, such as a smart phone with close-in communication software, which may transmit this data to a hospital or other location for monitoring of the patient and cast 10. Optionally, the cast 10 may function without a sleeve 20. Optionally, the sleeve 20 comprises a flexible compression-sock fabric.

Fig. 17 illustrates a system overview of the cast 10 for saving and communicating sensor data, in accordance with an embodiment of the present invention. A processor 110, a sensor module 140, a memory module 150, and a communication module 160 are connected by one or more control and/or data buses. The control and/or data buses are shown generally in Fig. 17 for illustrative purposes. The sensor module 140 includes at least one sensor 40 as described above. The sensors 40 of the sensor module 140 may be organized in a variety of iterations and configurations.

The processor 110 is operable to receive data from the sensor module 40, and may perform calculations and/or store the data in the memory module 150. The communication module 160 enables communication via a network to a portable device (such as, for example, a smart phone with close-in communication software). The communication module 160 may be, for example, but not limited to, a Wi-Fi chip, a Bluetooth chip, or a GSM chip. In some embodiments, a signal can be transmitted at real-time by the communication module 160 or stored in the memory unit 150 until a connection between the communication module 160 and the network is established. In other embodiments, Near Field Communication ("NFC") or infrared ("IR") technology is used to transmit signals using the communication module 160.

Data derived from monitoring bio-sensor feedback across a volume of users serves as a constantly updating normative baseline for casting metrics and then reconciled with the individual user's baseline to achieve an optimal fit, with proper pressure. An algorithm(s) is derived and serves as a proper casting standard and an early warning to complications of casting. An example would include, but not be limited to, compartment syndrome. The problems that would be addressed include the latitude and variability in traditional casting methods and lack of uniform standards, along with the resulting inconsistency and inability to predict complications. In a variant, the memory module 150 has computer readable instructions stored thereon configured to cause the processor 100 to receive data from the sensors 40 of the sensor module 140 to determine and monitor proper fit of the cast 10, progression of healing, and along with the patient's individual baselines normative sensor data, can issue an early warning of complications based on the objective.

In one example embodiment, once deployed, the cast 10 begins collecting data, and when the desired pressure is achieved and the cast 10 is hardened and set, the data set collected, along with normative data, and an algorithm, becomes the baseline for this specific incident. Once deployed, the sensors 40 continue monitoring for deviations from the baseline, so that the cast 10 can alert a monitoring practitioner and/or a user to problems. In one example, a combination of a decrease in blood oxygen levels at the distal casting point, a weaker pulse, lower blood pressure, decreased pH, and other bio-data that runs automatically through an algorithm, determines via the processor, the earliest possible point, whether there is a threat of compartment syndrome. Early recognition of compartment syndrome can reduce the likelihood of damage to nerves and/or muscles and/or other tissue during use of the cast 10.

Graphene wires 55 or spires are also housed within the bladder tubes 30 and are electrically conductive. The wires 55 may be graphene, graphene composite or graphene oxide or other electrically conductive materials. Electronic pulses or vibrations, for example, at low frequency, may be provided through the wires 55 within the bladder network 30 to promote healing of the broken bone. The low frequency may be, for example, but not limited to, 10 to 100 Hz and a plurality of durations and/or schedules for applying the low frequency are known in the art. The electronic pulses or vibrations at the low frequency stimulates the patient's osteoblasts to produce more bone material; other frequencies and effects may be known in the art.

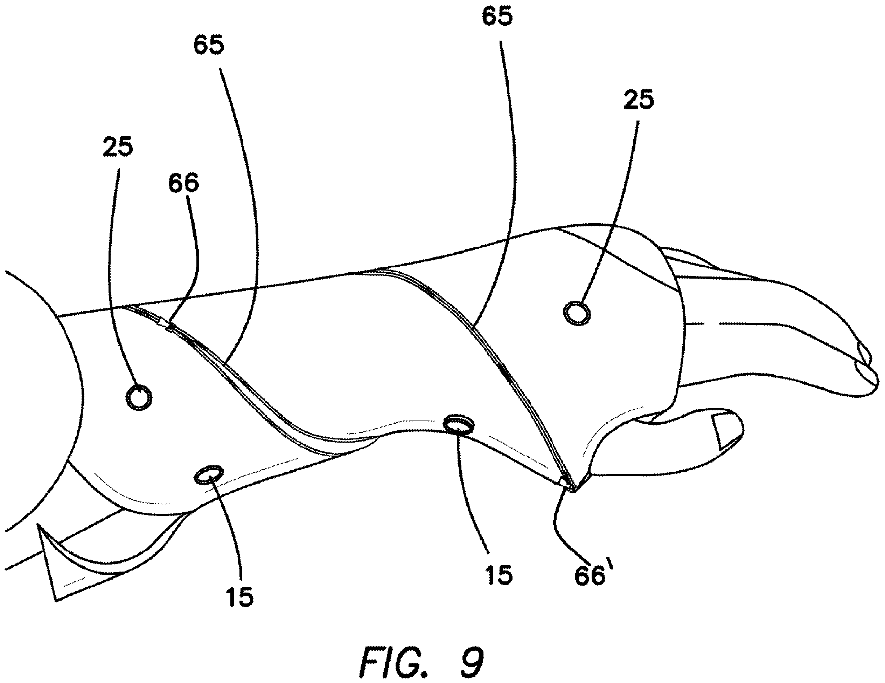

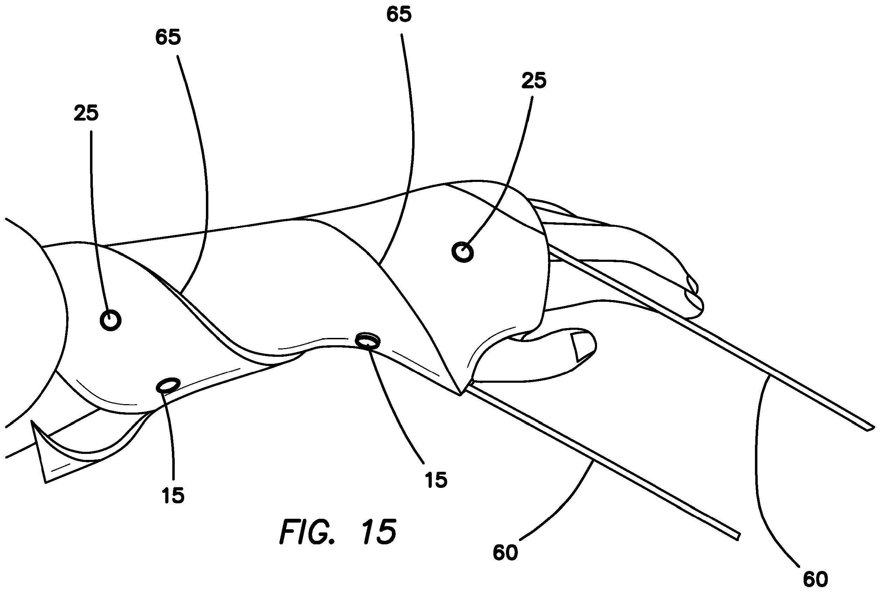

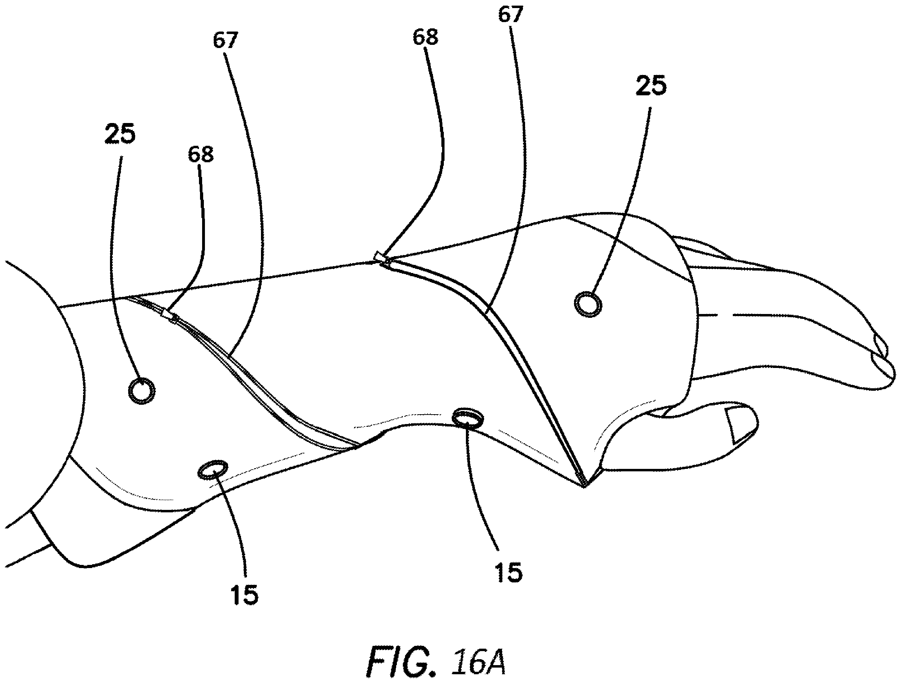

In a variant, referring to Fig. 9, to remove the cast 10, a zipper connection 66 may be configured to hold together or allow separation of the seams 65, thus providing a mechanism for removing the cast 10. The user pulls the zipper connection 66 to cause the cast 10 to separate along the seams 65, allowing the user to easily remove the cast 10. Zipper connection 66 is in a zipped and/or closed position while Zipper connection 66' is in an unzipped and/or open position. In an alternate variant, referring to Fig. 15, to remove the cast 10, the user pulls two or more wires 60 that cause the outer covering of the cast 10 to separate along seams 65. This is analogous to pulling a full-length-pin out of a very long, curved hinge. As a result, the cast 10 comes apart so that the user may easily remove it. A further variant of the cast 10 may include at least one expansion seam in parallel to the seams 65 (described below with respect to Figs. 16A-C).

In a variant, referring to Figs. 16A-C, to expand the cast 10 and relieve some pressure while maintaining a degree of rigidity and support, the user can unzip an expansion zipper 68 along at least one expansion seam 67, to allow the cast 10 to expand minimally along the expansion seams 67 like a suitcase that allows a traveler to pack additional clothing while maintaining integrity of the bag. The expansion zipper 68 may allow the cast 10 to expand, for example, but not limited to, less than 1 mm to reduce pressure and tension on the patient's limb.

Fig. 16A illustrates a cast 10 with the expansion zipper 68 unzipped. The expansion seams 67 are widened and an expansion material 69 covers the expanded area and connects parts of the cast 10. The expansion material 69 may be, for example, but not limited to, the same material as either the sleeve 10 or the cast 20. Other materials are possible and known in the art.

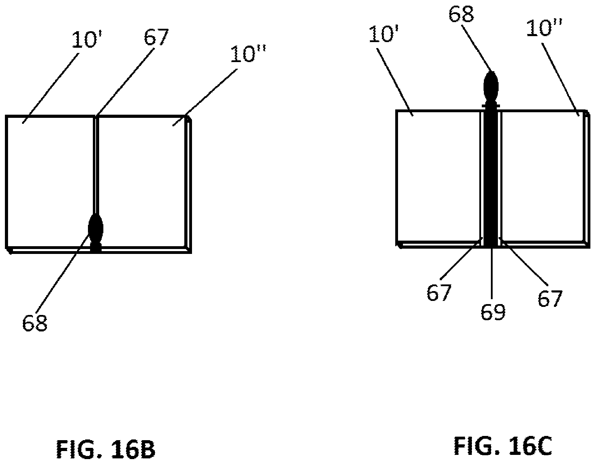

Fig. 16B illustrates a portion of the cast 10 before unzipping of the expansion zipper 68. Two parts of the cast 10' and 10" are held by the expansion seams 67. Fig. 16C illustrates a portion of the cast 10 after unzipping of the expansion zipper 68. Two parts of the cast 10' and 10" are held by the expansion material 69 between them. The expansion material 69 is attached between the expansion seams 67, and is stored underneath the seams 67 before unzipping of the expansion zipper 68. In some embodiments, the expansion seams 67 are parallel to the seams 65, illustrated in Figs. 9 and 15, used to remove the cast 10.

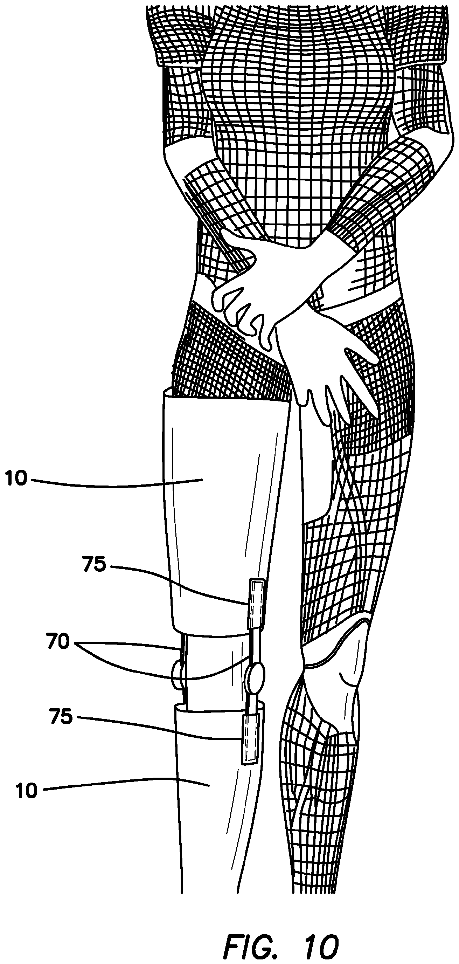

The cast 10 may be constructed in a number of alternative configurations, such as, for example illustrated in Fig. 10, a hinged knee brace 70 inserted in bilateral sleeves 75 between two casts 10 and/or a body cast 10 embodiment.

While various embodiments of the present invention have been described above, it should be understood that they have been presented by way of example only, and not of limitation. Likewise, the various diagrams may depict an example architectural or other configuration for the invention, which is done to aid in understanding the features and functionality that can be included in the invention. The invention is not restricted to the illustrated example architectures or configurations, but the desired features can be implemented using a variety of alternative architectures and configurations. Indeed, it will be apparent to one of skill in the art how alternative functional, logical or physical partitioning and configurations can be implemented to achieve the desired features of the present invention. Also, a multitude of different constituent module names other than those depicted herein can be applied to the various partitions. Additionally, with regard to flow diagrams, operational descriptions and method claims, the order in which the steps are presented herein shall not mandate that various embodiments be implemented to perform the recited functionality in the same order unless the context dictates otherwise.

Although the invention is described above in terms of various exemplary embodiments and implementations, it should be understood that the various features, aspects and functionality described in one or more of the individual embodiments are not limited in their applicability to the particular embodiment with which they are described, but instead can be applied, alone or in various combinations, to one or more of the other embodiments of the invention, whether or not such embodiments are described and whether or not such features are presented as being a part of a described embodiment. Thus the breadth and scope of the present invention should not be limited by any of the above-described exemplary embodiments.

Terms and phrases used in this document, and variations thereof, unless otherwise expressly stated, should be construed as open ended as opposed to limiting. As examples of the foregoing: the term "including" should be read as meaning "including, without limitation" or the like; the term "example" is used to provide exemplary instances of the item in discussion, not an exhaustive or limiting list thereof; the terms "a" or "an" should be read as meaning "at least one," "one or more" or the like; and adjectives such as "conventional," "traditional," "normal," "standard," "known" and terms of similar meaning should not be construed as limiting the item described to a given time period or to an item available as of a given time, but instead should be read to encompass conventional, traditional, normal, or standard technologies that may be available or known now or at any time in the future. Likewise, where this document refers to technologies that would be apparent or known to one of ordinary skill in the art, such technologies encompass those apparent or known to the skilled artisan now or at any time in the future.

A group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should also be read as "and/or" unless expressly stated otherwise. Furthermore, although items, elements or components of the invention may be described or claimed in the singular, the plural is contemplated to be within the scope thereof unless limitation to the singular is explicitly stated.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, can be combined in a single package or separately maintained and can further be distributed across multiple locations.

Additionally, the various embodiments set forth herein are described in terms of exemplary block diagrams, flow charts and other illustrations. As will become apparent to one of ordinary skill in the art after reading this document, the illustrated embodiments and their various alternatives can be implemented without confinement to the illustrated examples. For example, block diagrams and their accompanying description should not be construed as mandating a particular architecture or configuration.

## Claims

1. A quick deployment cast (10), comprising:
a flexible outer sleeve (20) fitted for a human appendage;
a bladder network (30) within the sleeve;
a fluid capable of expanding and hardening when in the presence of a curing agent, stored within the bladder network; and
a mechanism (15,18) for exposing the fluid to the curing agent;
**characterized in that** the quick deployment cast further comprises balloons (45) for receiving excess fluid during deployment of the cast.

2. The quick deployment cast of claim 1, wherein the pressure inside the interior space of the bladder network is at about vacuum.

3. The quick deployment cast of claim 1, wherein the fluid is polyurethane gel and the curing agent is air or a low volume of water.

4. The quick deployment cast of claim 1, wherein the fluid is polyurethane gel and the curing agent is a low volume of water contained within a sponge or on its own within a sealed parallel compartment, which combines with the polyurethane when a membrane is stretched and stressed to break, allowing the gel and the water to combine.

5. The quick deployment cast of claim 1, further comprising balloon ports (25) 2. on the outside of the sleeve for housing the balloons (45).

6. The quick deployment cast of claim 3, wherein the mechanism is configured for exposing the fluid to the air as the curing agent.

7. The quick deployment cast of any one of claims 1-5, further comprising a mechanism for removing the quick deployment cast after it has been deployed and its fluid cured, wherein the mechanism is selected from the group consisting of:
a wire (60) embedded within the cast (10), configured to be pulled out of the cast to cause the cast to separate at seams (65);
a zipper (66) configured to cause the cast to separate at seams (65) when unzipped; and
a zipper (68) configured to cause a controlled amount of separation and release of pressure while maintaining integrity, allowing the wearer to benefit from the stability of the cast.

8. The quick deployment cast of claim 1, further
comprising electrically conductive spires embedded in the bladder network; and
sensors (40) for detecting tension or pressure; wherein
the cast is configured such that when a predetermined tension on the bladder network is reached, excess fluid is directed into the balloons (45).

9. The quick deployment cast of claim 1, further sensors (40) configured to detect pressure against a limb, and to redirect flow of the fluid to the balloons when the cast is fully deployed and proper tension is achieved.

10. The quick deployment cast of claim 1, wherein the bladder network further comprises one or multiple intersecting sets of hollow ducts.

11. The quick deployment cast of claim 8, further comprising sensors (40) configured to monitor bio data, including but not limited to: pH, pulse, blood-flow, blood oxygen, enzymes and proteins, surface and blood pressure, relative positioning and potential deviation from a desired standard, and an electronic device capable of receiving and monitoring information from the sensors.

12. The quick deployment cast of claim 11, further comprising a (100) and a memory with computer readable instructions stored thereon and configured to cause the processor to receive data from the sensors and determine and monitor proper fit, progression of healing, and early warning of complications based on the sensor data.

13. The quick deployment cast of claim 12,
wherein
once deployed, the cast collects data forming a data set, until a predetermined pressure is achieved and the cast is hardened and set, and the data set is stored in the memory as a baseline; wherein
after the cast is set the sensors continue monitoring for deviations from the baseline and the cast generates an alert upon a detected deviation from the baseline data.

14. The quick deployment cast of claim 1, wherein the mechanism for exposing the fluid to the curing agent is a check valve port (15).

## Patentansprüche

1. Schnelleinsatzgips (10), Folgendes umfassend:
eine flexible äußere Hülse (20), geeignet für eine menschliche Gliedmaße;
ein Blasennetzwerk (30) innerhalb der Hülse;
ein Fluid, welches in der Lage ist, wenn es in der Präsenz eines Härtemittels ist, zu expandieren und zu härten, welches innerhalb des Blasennetzwerks gespeichert ist; und
einen Mechanismus (15,18) zum Exponieren des Fluids gegenüber dem Härtemittel;
**dadurch gekennzeichnet, dass** der Schnelleinsatzgips ferner Ballons (45) zum Aufnehmen von überschüssigem Fluid während des Einsatzes des Gipses umfasst.

2. Schnelleinsatzgips nach Anspruch 1, wobei der Druck innerhalb des Innenraums des Blasennetzwerks ungefähr Vakuumdruck beträgt.

3. Schnelleinsatzgips nach Anspruch 1, wobei das Fluid Polyurethangel ist und das Härtemittel Luft oder ein geringes Volumen an Wasser ist.

4. Schnelleinsatzgips nach Anspruch 1, wobei das Fluid Polyurethangel ist und das Härtemittel ein geringes Volumen an Wasser ist, welches innerhalb eines Schwamms oder allein innerhalb eines versiegelten parallelen Fachs enthalten ist, welches sich mit dem Polyurethan kombiniert, wenn eine Membran gestreckt und bis zum Bruch beansprucht wird, wodurch sie das Gel und das Wasser in die Lage versetzt, sich zu kombinieren.

5. Schnelleinsatzgips nach Anspruch 1, ferner umfassend Ballonanschlüsse (25) an der Außenseite der Hülse zur Unterbringung der Ballons (45).

6. Schnelleinsatzgips nach Anspruch 3, wobei der Mechanismus konfiguriert ist, um das Fluid gegenüber der Luft als dem Härtemittel zu exponieren.

7. Schnelleinsatzgips nach einem der Ansprüche 1 bis 5, ferner umfassend einen Mechanismus zum Entfernen des Schnelleinsatzgipses nachdem dieser eingesetzt wurde und sein Fluid gehärtet ist, wobei der Mechanismus aus der Gruppe ausgewählt ist, bestehend aus:
einem innerhalb des Gipses (10) eingebetteten Draht (60), welcher konfiguriert ist, um aus dem Gips herausgezogen zu werden, um den Gips dazu zu veranlassen, sich an Säumen (65) zu trennen;
einen Reißverschluss (66), welcher konfiguriert ist, um den Gips dazu zu veranlassen, sich an Säumen (65) zu trennen, wenn er geöffnet wird; und
einen Reißverschluss (68), welcher konfiguriert ist, um eine kontrollierte Menge an Trennung und Druckentlastung zu verursachen, während er die Integrität erhält, wodurch der Träger in die Lage versetzt wird, Nutzen aus der Stabilität des Gipses zu ziehen.

8. Schnelleinsatzgips nach Anspruch 1, zudem Folgendes umfassend:
elektrisch leitfähige Windungen, welche in dem Blasennetzwerk eingebettet sind; und
Sensoren (40) zur Detektion von Spannung oder Druck; wobei der Gips dergestalt konfiguriert ist, dass, wenn eine vorbestimmte Spannung an dem Blasennetzwerk erreicht ist, überschüssiges Fluid in die Ballons (45) geleitet wird.

9. Schnelleinsatzgips nach Anspruch 1, zudem Folgendes umfassend:
Sensoren (40), welche konfiguriert sind, um Druck gegen eine Gliedmaße zu detektieren, und um eine Strömung des Fluids an die Ballons umzuleiten, wenn der Gips vollständig eingesetzt ist und eine geeignete Spannung erzielt ist.

10. Schnelleinsatzgips nach Anspruch 1, wobei das Blasennetzwerk ferner einen oder mehrere einander kreuzende Sätze von hohlen Kanälen umfasst.

11. Schnelleinsatzgips nach Anspruch 8, ferner umfassend Sensoren (40), welche konfiguriert sind, um biologische Daten zu überwachen, darin eingeschlossen jedoch nicht hierauf begrenzt: pH, Puls, Blutströmung, Blutsauerstoff, Enzyme und Proteine, Oberflächen- und Blutdruck, relative Positionierung und potentielle Abweichung von einem gewünschten Standard, und eine elektronische Vorrichtung, welche in der Lage ist, Informationen von den Sensoren zu empfangen und zu überwachen.

12. Schnelleinsatzgips nach Anspruch 11, ferner umfassend einen Prozessor (100) und einen Speicher mit computerlesbaren Anweisungen, welche darin gespeichert und konfiguriert sind, um den Prozessor zum Empfangen von Daten von den Sensoren und zum Bestimmen und Überwachen einer ordnungsgemäßen Passung, eines Heilungsfortschritts und früher Warnzeichen von Komplikationen, basierend auf den Sensordaten, zu veranlassen.

13. Schnelleinsatzgips nach Anspruch 12, wobei der Gips, sobald er eingesetzt ist, Daten sammelt, welche einen Datensatz bilden, bis ein vorbestimmter Druck erzielt ist und der Gips gehärtet ist und abgebunden hat, und der Datensatz in dem Speicher als eine Grundlinie gespeichert wird; wobei
nachdem der Gips abgebunden hat, die Sensoren weiterhin Abweichungen von der Grundlinie überwachen und der Gips einen Alarm bei einer detektierten Abweichung von den Grundliniendaten erzeugt.

14. Schnelleinsatzgips nach Anspruch 1, wobei der Mechanismus zum Exponieren des Fluids gegenüber dem Härtemittel ein Rückschlagventil-Anschluss (15) ist.

## Revendications

1. Plâtre à déploiement rapide (10), comprenant :
un manchon extérieur flexible (20) adapté pour un membre humain ;
un réseau de bulles (30) dans le manchon ;
un fluide capable de se dilater et de se durcir en présence d'un agent durcissant, stocké dans le réseau de bulles ; et
un mécanisme (15, 18) permettant d'exposer le fluide à un agent durcissant ;
**caractérisé en ce que** le plâtre à déploiement rapide comprend en outre des ballonnets (45) permettant de recevoir le fluide en excès pendant le déploiement du plâtre.

2. Plâtre à déploiement rapide selon la revendication 1, dans lequel la pression à l'intérieur de l'espace intérieur du réseau de bulles est à peu près celle du vide.

3. Plâtre à déploiement rapide selon la revendication 1, dans lequel le fluide est un gel de polyuréthane et l'agent durcissant est l'air ou un faible volume d'eau.

4. Plâtre à déploiement rapide selon la revendication 1, dans lequel le fluide est un gel de polyuréthane et l'agent durcissant est un faible volume d'eau contenu dans une éponge ou directement dans un compartiment parallèle scellé, qui se combine au polyuréthane lorsqu'une membrane est étirée et contrainte jusqu'à la rupture, permettant au gel et à l'eau de se combiner.

5. Plâtre à déploiement rapide selon la revendication 1, comprenant en outre des orifices de ballonnets (25) à l'extérieur du logement permettant d'abriter les ballonnets (45).

6. Plâtre à déploiement rapide selon la revendication 3, dans lequel le mécanisme est configuré pour exposer le fluide à l'air en tant qu'agent durcissant.

7. Plâtre à déploiement rapide selon l'une quelconque des revendications 1 à 5, comprenant en outre un mécanisme permettant d'enlever le plâtre à déploiement rapide après son déploiement et une fois son fluide durci, dans lequel le mécanisme est sélectionné dans le groupe constitué de :
un fil (60) intégré dans le plâtre (10), configuré pour être tiré hors du plâtre afin de provoquer la séparation du plâtre au niveau de coutures (65) ;
une fermeture éclair (66) configurée pour provoquer la séparation du plâtre au niveau de coutures (65) lorsqu'elle est dézippée ; et
une fermeture éclair (68) configurée pour provoquer une quantité contrôlée de séparation et libérer une pression tout en maintenant l'intégrité, afin de permettre au porteur de bénéficier de la stabilité du plâtre.

8. Plâtre à déploiement rapide selon la revendication 1, comprenant en outre :
des spires électriquement conductrices intégrées dans le réseau de bulles ; et
des capteurs (40) permettant de détecter la tension ou la pression ; dans lequel le plâtre est configuré de sorte que lorsqu'une tension prédéterminée sur le réseau de bulles est atteinte, le fluide en excès est dirigé dans les ballonnets (45).

9. Plâtre à déploiement rapide selon la revendication 1, comprenant en outre des capteurs (40) configurés pour détecter une pression contre un membre et pour rediriger le flux de fluide vers les ballonnets quand le plâtre est totalement déployé et qu'une tension correcte est atteinte.

10. Plâtre à déploiement rapide selon la revendication 1, dans lequel le réseau de bulles comprend en outre un ou plusieurs ensembles s'entrecoupant de conduites creuses.

11. Plâtre à déploiement rapide selon la revendication 8, comprenant en outre des capteurs (40) configurés pour contrôler des biodonnées, y compris, sans y être limités : le pH, le pouls, le débit sanguin, l'oxygène du sang, les enzymes et les protéines, la pression de surface et sanguine, le positionnement relatif et l'écart potentiel par rapport à une norme souhaitée, et un dispositif électronique capable de recevoir et de surveiller les informations provenant des capteurs.

12. Plâtre à déploiement rapide selon la revendication 11, comprenant en outre un processeur (100) et une mémoire avec des instructions lisibles sur ordinateur stockées à l'intérieur, et configurée pour amener le processeur à recevoir des données des capteurs et à déterminer et à contrôler l'ajustement adéquat, la progression de la guérison et les signes annonciateurs de complications basées sur les données du capteur.

13. Plâtre à déploiement rapide selon la revendication 12, dans lequel une fois déployé, le plâtre collecte des données formant un ensemble de données, jusqu'à ce qu'une pression prédéterminée soit atteinte et que le plâtre soit durci et fixé, et que l'ensemble de données soit stocké dans la mémoire comme base de référence ; dans lequel
une fois le plâtre durci, les capteurs continuent à surveiller les écarts par rapport à la base de référence et le plâtre génère une alerte en cas d'écart détecté par rapport aux données de la base de référence.

14. Plâtre à déploiement rapide selon la revendication 1, dans lequel le mécanisme permettant d'exposer le fluide à l'agent durcissant est un orifice de clapet de retenue (15).
